# EUROPÄISCHE PATENTSCHRIFT

(11) **EP 2 607 894 B1**
(45) Veröffentlichungstag und Bekanntmachung des Hinweises auf die Patenterteilung: **07.10.2015**
(21) Anmeldenummer: 11010155.7
(22) Anmeldetag: 23.12.2011
(51) Int. Cl.: G01N 29/14, G01N 29/22, G01N 29/27, G01N 29/44, B27D 1/04, G01N 33/46

(54) **Verfahren und Vorrichtung zur zerstörungsfreien Körperschallprüfung von Holzwerkstoffplatten**
Method and apparatus for non-destructive acoustic emission inspection of wood boards
Procédé et dispositif de contrôle non destructif de plaques en matériaux dérivés du bois par émission acoustique

(43) Veröffentlichungstag der Anmeldung: 26.06.2013
(73) Patentinhaber: Kronotec AG, 6006 Luzern (CH)
(72) Erfinder:
(74) Vertreter: Rehmann, Thorsten

(56) Entgegenhaltungen:
- WO-A1-2010/009750
- DE-C1- 4 406 385
- JP-A- 2 269 963
- US-A- 4 831 880
- US-A- 6 029 522
- YU I MEREM'YANIN: "ACOUSTIC MEASUREMENT OF THE WATER CONTENT OF MOVING WOOD SHAVINGS", MEASUREMENT TECHNIQUES, CONSULTANTS BUREAU. NEW YORK, US, Bd. 29, Nr. 4, 1. April 1986 (1986-04-01), Seiten 354-355, XP001330961, ISSN: 0543-1972

## Beschreibung

Die Erfindung betrifft ein Verfahren zur zerstörungsfreien Überprüfung der Güte von Holzwerkstoffplatten, die als reproduzierbarer Plattentyp durch Verpressen von mit einem Klebstoff beleimten Holzschnitzeln, Spänen oder Fasern in einer Presse hergestellt wurden und in einer mit einem Transportgestell versehenen Transporteinrichtung in einer Transportrichtung transportiert werden, mittels Schallmessung.

Ein Verfahren zur zerstörungsfreien Überprüfung von Holzwerkstoffplatten mit einer entsprechend ausgestalteten Vorrichtung ist beispielsweise aus der DE 37 42 844 C2 oder der DE 44 06 385 C1 bekannt. Zur Qualitätssicherung von plattenförmigen Werkstücken, wie Span-, Faser- und/oder Sperrholzplatten, Holzzement- oder Holzgipsplatten ist es bekannt diese nach der Produktion von einem oder mehreren Ultraschall-Sendern zu durchstrahlen, um innenliegende Produktionsfehler, wie mangelnde Verleimung, Leimklumpen oder Steine zu entdecken. Dabei werden die Werkstücke in der Regel mittels Band- oder Rollentransport durch einen Messrahmen hindurchtransportiert, der mit der Messeinrichtung bestückt ist. Den Ultraschall-Sendern gegenüberliegend sind Empfänger angeordnet und die zu prüfende Platte wird zwischen dem Sender und dem Empfänger hindurchgefördert. Die Ankopplung an die zu prüfende Platte erfolgt über den Luftspalt zwischen Sender und Platte bzw. Empfänger und Platte. Befindet sich in der Platte innerhalb des Ultraschall-Feldes eine Fehlstelle, wird das Empfangssignal verstärkt oder geschwächt.

In der JP 2 269963 A ist eine Sperrholz-Prüfvorrichtung beschrieben, bei der mittels eines Schallemissionssensors die in der Platte entstehende Schallemission erfasst wird. Die Platten werden durch einen zwischen zwei Rollen eingestellten Spalt hindurchgefördert und die Schallemissionen können dann durch einen Schallsensor detektiert werden, der an der oberen Anpressrolle befestigt ist.

In der US 4 831 880 A wird ein Verfahren und eine Vorrichtung zur vertikalen Dichtemessung von Platten beschrieben, bei der durch einen Schlagfräser ein vertikales Loch in die zu prüfende Platte gefräst wird und anhand der dabei erzeugten Lärmemission Rückschlüsse auf das Dichteprofil der Platte gezogen werden können.

Die WO 2010/009750 A1 beschreibt ein Verfahren und eine Anordnung zum Ermitteln und Überwachen des Zustands eines Wälzlagers. In diesem Verfahren werden ein erstes Sensorsignal in einem ersten Frequenzband und ein zweites Sensorsignal in einem zweiten Frequenzband erfasst. Aus der Signalform des ersten Sensorsignals wird ein erster Kennwert für eine gerade erfolgte Schädigung des Wälzlagers ermittelt. Aus der Signalform des zweiten Sensorsignals wird ein zweiter Kennwert für eine bereits erfolgte Schädigung des Wälzlagers ermittelt.

Neben den Fehlern, die innerhalb der Platte vorhanden sind (zum Beispiel Spalter = Verleimfehler oder Leimklumpen oder dergleichen) wird die Qualität einer Platte aber auch durch Schwankungen bei den Einsatzstoffen selbst und/oder technologisch bedingten Produktionsschwankungen beeinträchtigt. Um auszuschließen, dass z. B. die Dicke einer verpressten Platte nicht dem Sollmaß entspricht, wird deshalb immer mit Sicherheitszulagen produziert. Diese Sicherheitszulagen verteuern das Produkt.

Oft werden solche Qualitätsmängel erst bei der Weiterverarbeitung aufgedeckt, sodass sich Verluste und Mehrkosten bei der Herstellung signifikant erhöhen. Es besteht deshalb ein Bedarf an einer zerstörungsfreien Prüfung, die in dem Produktionsprozess integrierbar ist und die Plattenqualität online bewerten kann, damit etwaige Qualitätsfehler der Platte möglichst frühzeitig aufgedeckt werden.

Der Erfindung liegt die Aufgabe zu Grunde, ein Verfahren anzugeben, mit dem kostengünstig innerhalb des Produktionsprozesses festgestellt werden kann, ob die produzierte Platte die geforderten Qualitätskriterien erfüllt, das einfach in den bestehenden Fertigungsprozess integrierbar ist und sich auf unterschiedliche Plattentypen anpassen lässt. Außerdem soll eine Vorrichtung zur Verwendung in dem Verfahren zur Verfügung gestellt werden.

Die Aufgabe wird bei dem eingangs erläuterten Verfahren erfindungsgemäß dadurch gelöst, dass
a) zur Ermittlung eines Referenz-Frequenzbandes für einen speziellen Plattentyp, ein Körperschall, den das von einer Platte dieses Plattentyps, die die geforderten Qualitätskriterien erfüllt, während ihres Transportes auf dem Weg durch den Fertigungsprozess angeregte Transportgestell abgibt, an einem räumlich definierten Ort detektiert wird,
b) das Referenz-Frequenzband als Sollwert in einer elektronischen Datenverarbeitungsanlage gespeichert wird,
c) das Frequenzband des Körperschalls, den das von der zu überprüfenden Platte desselben Plattentyps angeregte Transportgestell abgibt, am selben definierten Ort ermittelt wird,
d) das Frequenzband als Ist-Wert in der elektronischen Datenverarbeitungsanlage gespeichert wird,
e) von der Datenverarbeitungsanlage der Ist-Wert mit dem Soll-Wert verglichen wird und
f) bei einer vorgegebenen Abweichung des Ist-Wertes vom Soll-Wert ein Signal erzeugt wird.

Der Erfindung liegt die Erkenntnis zu Grunde, dass die Holzwerkstoffplatte auf ihrem weiteren Transport durch den Fertigungs- bzw. Bearbeitungsprozess in Schwingungen versetzt wird, die sie auf alle die Bauteile überträgt, mit denen sie in Berührung kommt. Auch diese Bauteile beginnen zu schwingen und an einer bestimmten Stelle kann dann der Körperschall eines von der Platte in Schwingung versetzten Bauteiles durch Detektierung eines Frequenzbandes ermittelt werden.

Der Körperschall des in Schwingung gesetzten Bauteiles an sich ist kein Maß für die Qualität der Platte. Wird aber der Körperschall ermittelt, wenn eine Platte, die in ihren technologischen Werten (Typ, Dicke, Festigkeit, Länge, Breite usw.) dem Qualitätsmaßstab entspricht, als Referenz-Frequenzband ermittelt und anschließend der Körperschall an demselben Ort von jeder Platte desselben Plattentyps, die produziert wurde und denselben technologischen Werten entsprechen muss, ermittelt wird, kann das Referenz-Frequenzband mit dem aktuellen Frequenzband verglichen werden. Eine Abweichung, die über ein zuvor festgelegtes Maß hinausgeht, zeigt an, dass diese Platte den Qualitätsanforderungen nicht entspricht. Es kann dann sofort überprüft werden, welcher Parameter nicht eingehalten wurde und die Produktion entsprechend "nachreguliert" werden. Durch die sofortige Auswertung des Frequenzbandes lässt sich die Plattenqualität der aktuell produzierten Platte also ermitteln und ein Operator kann sofort eingreifen. Fehlchargen lassen sich deshalb sicher detektieren und deren Weiterverarbeitung somit sicher vermeiden.

In Versuchen wurde festgestellt, dass bei steigenden Festigkeiten spezifische Frequenzen des detektierten Frequenzbandes verstärkt sind und sinkende Festigkeiten einen Abfall dieser Frequenzen bedeuten.

Der Körperschall des Transportgestells, über das die Platte transportiert wird, wird detektiert. Das Transportgestell kommt mit der heißen Platte nicht direkt in Berührung. Die Temperatur des Gestells liegt folglich allenfalls nur geringfügig über der Raumtemperatur. Dadurch ist es möglich, den Ort der Ermittlung für das Frequenzband möglichst dicht hinter die Heißpresse zu legen, um sehr frühzeitig die nicht akzeptable Qualität der soeben produzierten Platte zu ermitteln, ohne auf teuere hitzefeste Sensoren zurückgreifen zu müssen.

Zur Detektion des Körperschalles ist ein Schallaufnehmer vorgesehen, der handelsüblich sein kann.

Vorzugsweise wird die Platte auf einem Transportrollengang aus der Presse heraus transportiert, sodass der Körperschall am Gestell des Transportrollenganges ermittelbar ist.

Die Vorrichtung zur Ermittlung des Körperschalls zur Verwendung in dem zuvor beschriebenen Verfahren weist also zumindest auf:
- eine Transporteinrichtung zum Transportieren einer darauf aufliegenden Platte,
   -- die Transporteinrichtung weist ein Gestell auf,
   -- das Gestell weist eine Mehrzahl in ihm drehbar gelagerte Rollen auf, die zueinander beabstandet und zum Tragen der Platte vorgesehen sind,
- einen an dem Gestell befestigten Schallaufnehmer zum Detektieren des von dem Gestell beim Transport einer Platte abgegebenen Körperschalls,
- eine elektronische Datenverarbeitungsanlage, die dazu eingerichtet ist, folgende Schritte durchzuführen:
   a) Ermittlung eines Referenz-Frequenzbandes für einen speziellen Plattentyp aus dem Körperschall, den das von der Platte dieses Plattentyps, die die geforderten Qualitätskriterien erfüllt, während ihres Transportes auf dem Weg durch den Fertigungsprozess angeregte Gestell abgibt,
   b) Speicherung des Referenz-Frequenzbandes als Soll-Wert,
   c) Speicherung des Frequenzbandes des Körperschalls, den das von der zu überprüfenden Platte desselben Plattentyps angeregte Gestell am selben definierten Ort abgibt, als Ist-Wert,
   d) Vergleichen des Ist-Werts mit dem Soll-Wert,
- eine mit der Datenverarbeitungsanlage verbundene Signaleinrichtung, die bei einer vorgegebenen Abweichung des Ist-Wertes vom Soll-Wert ein Signal erzeugt.

Der Schallaufnehmer ist vorzugsweise über einen Analog-/Digital-Wandler mit der elektronischen Datenverarbeitungsanlage verbunden.

Das Frequenzband kann ein Oktavband oder auch ein Terzband oder jedes andere Frequenzband sein.

Mit Hilfe einer Zeichnung soll ein Ausführungsbeispiel der Erfindung nachfolgend näher beschrieben werden.

Es zeigen:

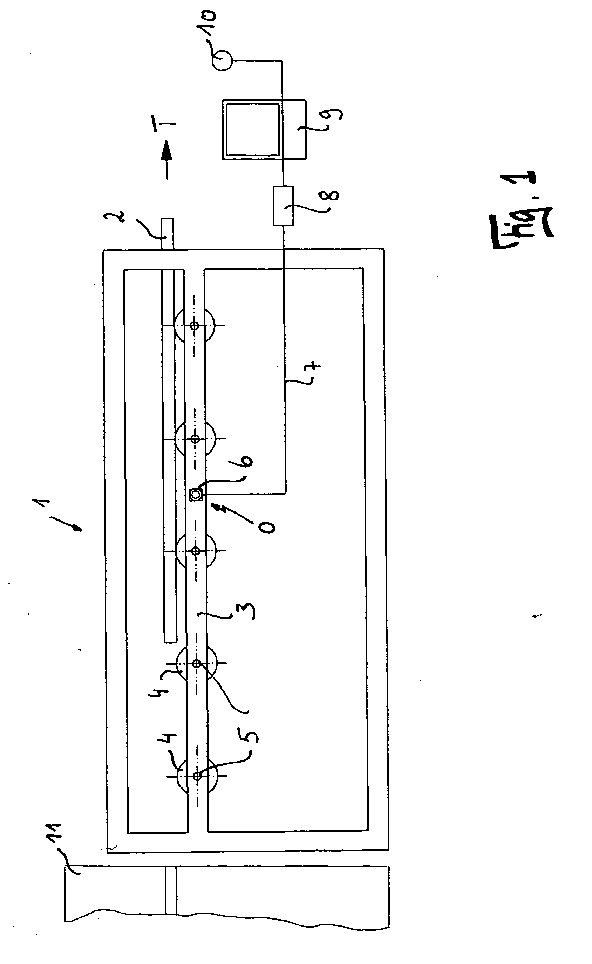

- Figur 1 -: die schematische Darstellung der Transporteinrichtung,
- Figur 2 -: ein Referenz-Oktavband,

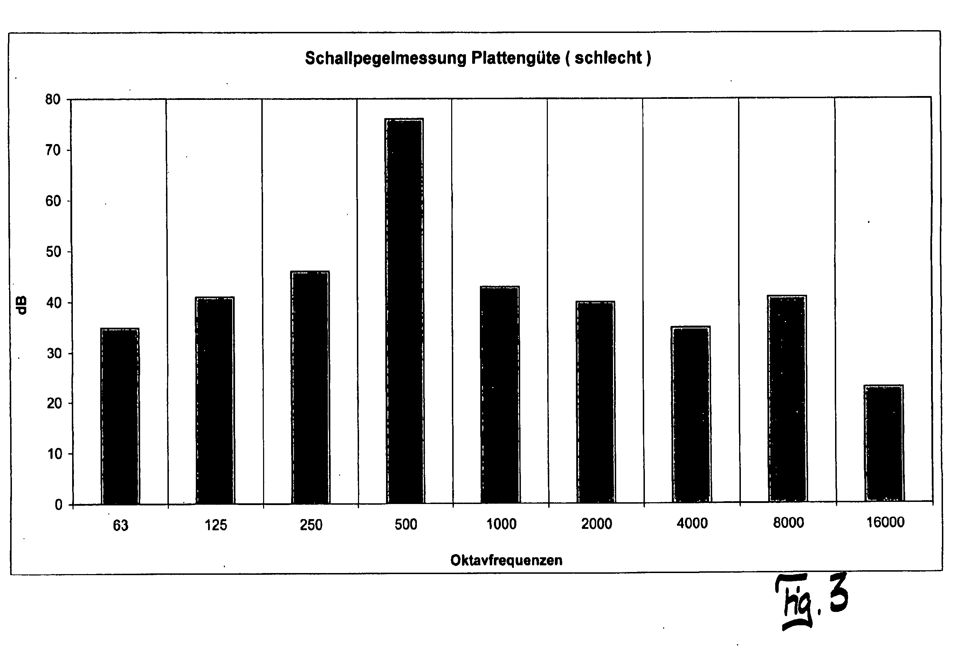

- Figur 3 -: ein Oktavband,

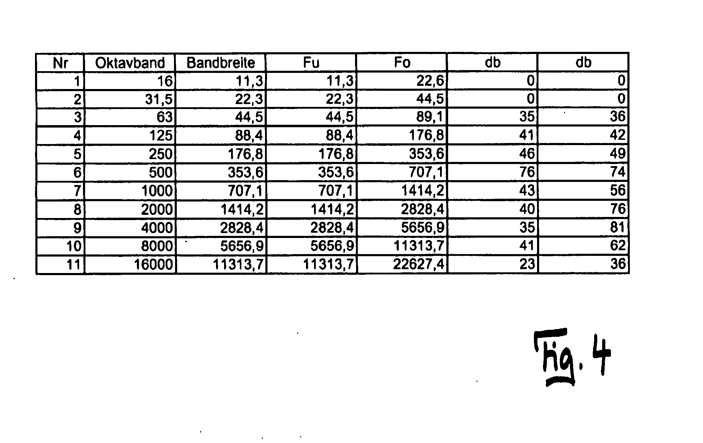

- Figur 4 -: die tabellarische Darstellung eines Oktavbandes.

Die Transporteinrichtung 1 ist ein sogenannter Transportrollengang und ist unmittelbar hinter der Heißpresse 11 angeordnet, aus der die gepresste Holzwerkstoffplatte 2 in Transportrichtung T herausgefördert wird. Die Transporteinrichtung 1 weist eine Mehrzahl von drehbar in dem Gestell 3 gelagerten Rollen 4 auf, deren Achsen 5 zueinander vorzugsweise parallel beabstandet sind. Am Gestell 3 ist ein Schallaufnehmer 6 befestigt, der über die Signalleitung 7 und einem Analog/Digital-Wandler 8 mit der elektronischen Datenverarbeitungseinrichtung 9 verbunden ist.

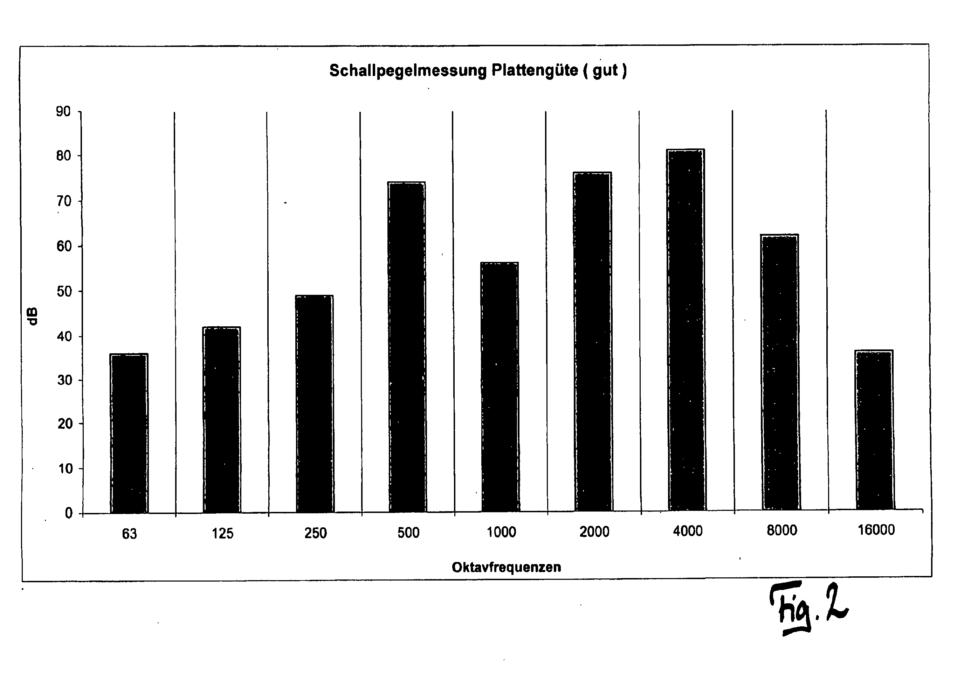

Zur Aufnahme des in Figur 2 gezeigten Referenz-Oktavbandes wird zunächst eine Holzwerkstoffplatte 2, die den Qualitätsanforderungen, der an den bestimmten Plattentyp (Dicke, Material, Breite, Länge, Dichte usw.) gestellt wird, in jeder Hinsicht entspricht, mittels der Transporteinrichtung 1 am Schallaufnehmer 6 vorbeigeführt. Während die Platte 2 den Schallaufnehmer 6 passiert, wird das Frequenzband des Körperschalls, den das von der transportierten Platte 2 angeregte Gestell 3 abgibt, ermittelt und über den Analog/DigitalWandler 8 der Datenverarbeitungsanlage 9 zugeführt und dort gespeichert. Die Geschwindigkeit der Platte 2 in Transportrichtung T entspricht dabei der Geschwindigkeit, mit der die in der Presse 11 gepressten Platten 2 aus dieser herausgefördert werden.

In derselben Art und Weise wird anschließend jede in der Presse 11 neu gepresste Platte 2 über den Schallaufnehmer 6 hinweggefördert und dabei das Oktavband dieser Platte ermittelt und als Ist-Wert der Datenverarbeitungsanlage 9 zugeführt. In der Datenverarbeitungsanlage 9 wird der Ist-Wert sofort mit dem Soll-Wert verglichen und wenn eine vorgegebene Abweichung ermittelt wird, wird die Signaleinrichtung 10 aktiviert. Die Signaleinrichtung 10 kann eine einfache Leuchte sein, die dem Bedienpersonal anzeigt, dass eine Platte minderer Qualität ermittelt wurde. Die Signaleinrichtung kann aber auch eine Steuereinrichtung sein, die beispielsweise den weiteren Prozess stoppt, sodass die fehlerhafte Platte 2 nicht mehr weitertransportiert wird.

Das Bedienpersonal muss dann feststellen, warum die ermittelte Platte 2 der Sollvorgabe nicht entspricht. Dann kann es entscheiden, ob der Produktionsprozess in der Presse oder davor zu ändern ist oder die Abweichung der Platte noch toleriert werden kann, sodass eine Weiterverarbeitung möglich ist.

Das in den Figuren dargestellte Oktavband ist ein Beispiel dafür, wie ermittelte Frequenzwerte aufbereitet werden. Durch Clusterung, im Fall des Oktavbandes ist dies eine logarithmische Summierung, ist es möglich, visuelle Unterschiede schnell zu erfassen, was für eine Gut-Schlecht-Bewertung oder eine Schlecht-Gut-Bewertung vorteilhaft ist.

### Bezugszeichenliste

1 Transporteinrichtung
2 Platte/Holzwerkstoffplatte
3 Gestell
4 Rolle
5 Achse
6 Schallaufnehmer
7 Leitung
8 Analog-/Digital-Wandler
9 elektronische Datenverarbeitungsanlage
10 Signal- und/oder Steuereinrichtung
11 Heißpresse/Presse
O Ort
T Transportrichtung

## Patentansprüche

1. Verfahren zur zerstörungsfreien Überprüfung der Güte von Holzwerkstoffplatten, die als reproduzierbarer Plattentyp insbesondere durch Verpressen von mit einem Klebstoff beleimten Holzschnitzeln, Spänen oder Fasern in einer Presse (11) hergestellt wurden und in einer mit einem Transportgestell (3) versehenen Transporteinrichtung (1) in einer Transportrichtung T transportiert werden, mittels Schallmessung,
**dadurch gekennzeichnet, dass**
a) zur Ermittlung eines Referenz-Frequenzbandes für einen speziellen Plattentyp, ein Körperschall, den das von einer Platte (2) dieses Plattentyps, die die geforderten Qualitätskriterien erfüllt, während ihres Transportes auf dem Weg durch den Fertigungsprozess angeregte Transportgestell (3) abgibt, an einem räumlich definierten Ort (O) detektiert wird,
b) das Referenz-Frequenzband als Soll-Wert in einer elektronischen Datenverarbeitungsanlage (9) gespeichert wird,
c) das Frequenzband des Körperschalls, den das von der zu überprüfenden Platte (2) desselben Plattentyps angeregte Transportgestell (3) abgibt, am selben definierten Ort (O) ermittelt wird,
d) das Frequenzband als Ist-Wert in der elektronischen Datenverarbeitungsanlage (9) gespeichert wird,
e) von der Datenverarbeitungsanlage (9) der Ist-Wert mit dem Soll-Wert verglichen wird und
f) bei einer vorgegebenen Abweichung des Ist-Wertes vom Soll-Wert ein Signal erzeugt wird.

2. Verfahren nach Anspruch 1, **dadurch gekennzeichnet, dass** zur Detektion des Körperschalls ein Schallaufnehmer (6) vorgesehen ist.

3. Verfahren nach Anspruch 1, **dadurch gekennzeichnet, dass** der Körperschall dicht hinter der Presse (11) ermittelt wird.

4. Verfahren nach Anspruch 1, **dadurch gekennzeichnet, dass** die Platte (2) auf einem Transportrollengang (1) aus der Presse heraustransportiert wird.

5. Verfahren nach Anspruch 1, **dadurch gekennzeichnet, dass** der Körperschall am Gestell (3) des Transportrollenganges (1) ermittelt wird.

6. Verfahren nach Anspruch 1, **dadurch gekennzeichnet, dass** das Frequenzband ein Oktavband ist.

7. Vorrichtung zur Ermittlung des Körperschalls zur Verwendung in dem Verfahren nach einem der vorstehenden Ansprüche, zumindest aufweisend:
- eine Transporteinrichtung (1) zum Transportieren einer darauf aufliegenden Platte (2),
-- die Transporteinrichtung (1) weist ein Gestell (3) auf,
-- das Gestell (3) weist eine Mehrzahl in ihm drehbar gelagerte Rollen (4) auf, die zueinander beabstandet und zum Tragen der Platte (2) vorgesehen sind,
- einen an dem Gestell (3) befestigten Schallaufnehmer (6) zum Detektieren des von dem Gestell (3) beim Transport jeder Platte (2) abgegebenen Körperschalls,
- eine elektronische Datenverarbeitungsanlage (9), die dazu eingerichtet ist, folgende Schritte durchzuführen:
a) Ermittlung eines Referenz-Frequenzbandes für einen speziellen Plattentyp, aus dem Körperschall, den das von der Platte (2) dieses Plattentyps, die die geforderten Qualitätskriterien erfüllt, während ihres Transportes auf dem Weg durch den Fertigungsprozess angeregte Gestell (3) abgibt,
b) Speicherung des Referenz-Frequenzbandes als Soll-Wert,
c) Speicherung des Frequenzbandes des Körperschalls, den das von der zu überprüfenden Platte (2) desselben Plattentyps angeregte Gestell (3) am selben definierten Ort (O) abgibt, als Ist-Wert,
d) Vergleichen des Ist-Werts mit dem Soll-Wert,
- eine mit der Datenverarbeitungsanlage (9) verbundene Signaleinrichtung (10), die bei einer vorgegebenen Abweichung des Ist-Wertes vom Soll-Wert ein Signal erzeugt.

8. Vorrichtung nach Anspruch 7, **dadurch gekennzeichnet, dass** der Schallaufnehmer (6) über einen Analog-/Digital-Wandler (8) mit der elektronischen Datenverarbeitungsanlage (9) verbunden ist.

## Claims

1. A method for non-destructive inspection of the quality of wood-based material boards, which were produced as a reproducible board type in particular by the pressing of wood chips, shavings or fibres, glued with an adhesive, in a press (11) and are transported in a transport arrangement (1), provided with a transport, frame (3), in a transport direction T, by means of acoustic measurement,
**characterized in that**
a) for determining a reference frequency band for a particular board type, a structure-borne sound, which the transport frame (3) emits, stimulated by a board (2) of this board type, which fulfils the required quality criteria, during its transportation on the way through the manufacturing process, is detected at a spatially defined location (0),
b) the reference frequency band is stored as the desired value in an electronic data processing system (9),
c) the frequency band of the structure-borne sound, which the transport frame (3) emits, stimulated by the board (2), to be inspected, of the same board type, is determined at the same defined location (0),
d) the frequency band is stored as an actual value in the electronic data processing system (9),
e) the actual value is compared by the data processing system (9) with the desired value and
f) in the case of a predetermined deviation of the actual value form the desired value, a signal is generated.

2. The method according to Claim 1, **characterized in that** a sound sensor (6) is provided for the detection of the structure-borne sound.

3. The method according to Claim 1, **characterized in that** the structure-borne sound is determined closely behind the press (11).

4. The method according to Claim 1, **characterized in that** the board (2) is transported out from the press on a transport roller conveyor (1).

5. The method according to Claim 1, **characterized in that** the structure-borne sound is determined on the frame (3) of the transport roller conveyor (1).

6. The method according to Claim 1, **characterized in that** the frequency band is an octave band.

7. A device for determining the structure-borne sound for use in the method according to one of the preceding claims, at least having:
- a transport arrangement (1) for transporting a board (2) resting thereon,
-- the transport arrangement (1) has a frame (3),
-- the frame (3) has a plurality of rollers (4) rotatably mounted in it, which are provided spaced apart from one another and for carrying the board (2),
- a sound sensor (6), fastened to the frame (3), for detecting the structure-borne sound emitted by the frame (3) on transportation of each board (2),
- an electronic data processing system (9), which is set up to carry out the following steps:
a) determining a reference frequency band for a particular board type, from the structure-borne sound which the frame (3) emits, stimulated by the board (2) of this board type, which fulfils the required quality critera, during its transportation on the way through the manufacturing process,
b) storing the reference frequency band as the desired value,
c) storing the frequency band of the structure-borne sound which the frame (3) emits, stimulated by the board (2) which is to be inspected, of the same board type, at the same defined location (0), as an actual value,
d) comparing the actual value with the desired value,
- a signal arrangement (10), connected with the data processing system (9), which signal arrangement generates a signal in the case of a predetermined deviation of the actual value from the desired value.

8. The device according to Claim 7, **characterized in that** the sound sensor (6) is connected with the electronic data processing system (9) via an analogue-to-digital converter (8).

## Revendications

1. Procédé de contrôle non destructif pour le contrôle de la qualité de panneaux en matériau à base de bois, qui ont été fabriqués en tant que type de panneaux reproductibles en particulier par compression dans une presse (11) de particules de bois, de copeaux ou de fibres encollé(e)s avec un adhésif et sont transportés dans une direction de transport T dans un dispositif de transport (1) muni d'un châssis de transport (3), par mesure acoustique,
**caractérisé en ce que**
a) pour la détermination d'une bande de fréquence de référence pour un type de panneaux spécifique, détecter en un lieu (O) défini dans l'espace un bruit de structure délivré par le châssis de transport (3) stimulé par un panneau (2), de ce type de panneaux, qui remplit les critères de qualité requis, pendant son transport sur le trajet à travers le processus de production,
b) enregistrer la bande de fréquence de référence comme valeur de consigne dans un système de traitement de données électronique (9),
c) déterminer sur le même lieu (O) défini la bande de fréquence du bruit de structure délivré par le châssis de transport (3) stimulé par le panneau (2) à contrôler du même type de panneaux,
d) enregistrer la bande de fréquence comme valeur réelle dans le système de traitement de données électronique (9),
e) par le système de traitement de données (9) comparer la valeur réelle avec la valeur de consigne et
f) générer un signal lors d'un écart prédéterminé de la valeur réelle par rapport à la valeur de consigne.

2. Procédé selon la revendication 1, **caractérisé en ce qu'**un capteur acoustique (6) est prévu pour la détection du bruit de structure.

3. Procédé selon la revendication 1, **caractérisé en ce que** le bruit de structure est déterminé juste derrière la presse (11).

4. Procédé selon la revendication 1, **caractérisé en ce que** le panneau (2) est extrait de la presse sur un convoyeur de transport à rouleaux (1).

5. Procédé selon la revendication 1, **caractérisé en ce que** le bruit de structure est déterminé sur le châssis (3) du convoyeur de transport à rouleaux (1).

6. Procédé selon la revendication 1, **caractérisé en ce que** la bande de fréquence est une bande d'octave.

7. Dispositif pour la détermination du bruit de structure destiné à être utilisé dans le procédé selon l'une quelconque des revendications précédentes, comprenant au moins :
- un dispositif de transport (1) pour le transport d'un panneau (2) posé dessus,
o le dispositif de transport (1) présente un châssis (3),
o le châssis (3) présente une pluralité de rouleaux (4) montés rotatifs sur celui-ci, les rouleaux étant espacés les uns par rapport aux autres et prévus pour porter le panneau (2),
- un capteur acoustique (6) fixé sur le châssis (3) pour détecter le bruit de structure délivré par le châssis (3) lors du transport de chaque panneau (2),
- un système de traitement de données électronique (9), conçu pour réaliser les étapes suivantes :
a) déterminer une bande de fréquence de référence pour un type de panneaux spécifique, à partir d'un bruit de structure délivré par le châssis (3) stimulé par le panneau (2) de ce type de panneaux qui remplit les critères de qualité requis, pendant son transport sur le trajet à travers le processus de production,
b) enregistrer la bande de fréquence de référence comme valeur de consigne,
c) enregistrer comme valeur réelle la bande de fréquence du bruit de structure que délivre au même lieu défini (O) le châssis (3) stimulé par le panneau (2) à contrôler du même type de panneaux,
d) comparer la valeur réelle avec la valeur de consigne,
- un dispositif de signalisation (10) relié au système de traitement de données (9) et qui génère un signal lors d'un écart prédéterminé de la valeur réelle par rapport à la valeur de consigne.

8. Dispositif selon la revendication 7, **caractérisé en ce que** le capteur acoustique (6) est relié au système de traitement de données électronique (9) par l'intermédiaire d'un convertisseur analogique/numérique (8).
